# EUROPÄISCHE PATENTSCHRIFT

(11) **EP 3 233 212 B1**
(45) Veröffentlichungstag und Bekanntmachung des Hinweises auf die Patenterteilung: **08.01.2020**
(21) Anmeldenummer: 15763505.3
(22) Anmeldetag: 25.08.2015
(51) Int. Cl.: A61Q 19/10, A61K 8/41, A61K 8/42, A61K 8/46, A61K 8/81, A61K 8/92, A61K 8/02, A61K 8/60, A61K 8/20, A61Q 19/00, A61K 8/34

(54) **PEELENDES REINIGUNGSPRODUKT AUF ÖLBASIS**
PEELING CLEASING PRODUCT ON THE BASIS OF OIL
COMPOSITION ABRASIVE À NETTOYAGE SUR LA BASE DE L'HUILE

(30) Priorität: 29.10.2014 DE 102014222014
(43) Veröffentlichungstag der Anmeldung: 25.10.2017
(73) Patentinhaber: Beiersdorf AG, 20253 Hamburg (DE)
(72) Erfinder: ARGEMBEAUX, Horst, 21465 Wentorf (DE); HOFF, Anke, 22589 Hamburg (DE)
(86) Internationale Anmeldenummer: PCT/EP2015/069386
(87) Internationale Veröffentlichungsnummer: WO 2016/066299

(56) Entgegenhaltungen:
- EP-A1- 1 967 173
- WO-A1-2014/190989

## Beschreibung

Die vorliegende Erfindung beschreibt Reinigungsprodukte auf Ölbasis, die abrasive Partikel, in einer besonderen Ausführungsform Salz- und/oder Zuckerkristalle, als peelende Substanzen, enthalten.

Die erfindungsgemäßen Öle sind Lipide, die sowohl Fette, die bei 20ºC fest oder halbfest sind, oder Öle, die flüssig sind, sein können. Native Öle und Fette sind chemisch im Wesentlichen Triacylglycerole höherer Fettsäuren mit gerader Anzahl von Kohlenstoffatomen. Petrochemische Öle oder Fette sind in der Regel längerkettige, mehr oder weniger verzweigte oder vernetzte, gesättigte oder ungesättigte oder teilgesättigte Kohlenwasserstoffe und deren Derivate.

Im weiteren Sinn wird unter dem Begriff Lipid eine bestimmte, strukturell an sich durchaus nicht einheitliche Gruppe von Biomolekülen verstanden, die ganz oder zumindest größtenteils wasserunlöslich ist.

Lipide im Sinne der vorliegenden Erfindung können beliebige Öle und/oder Fette der beschriebenen Form sein. Erfindungsgemäß sind es Mischungen von bei Raumtemperatur flüssigen und festen Lipiden.

Reinigungsprodukte, die einen erhöhten Öl- bzw. Lipidanteil enthalten, sind schon längere Zeit auf dem Markt. Das Öl in dem Reinigungsprodukt soll auf die Haut aufgebracht werden und die Haut schützen, den Hautzustand normalisieren und zur Regeneration von angegriffener, sensibler oder auch Altershaut beitragen.

Um die Schutzfunktion von Lipiden zu erklären, wurde folgende Vorstellung entwickelt: die Tenside, insbesondere anionische Tenside, können an der oberen Hautschicht adsorbieren und dort ihre Wirkung entfalten. Sie lösen einerseits Schmutzpartikel oder einen Schmutzfilm von der Haut. Jedoch wird nicht nur der Schmutz abgelöst, sondern andererseits werden auch hauteigene Lipide und natürliche Feuchthaltefaktoren (NMF, Natural Moisturizing Factors) mit abgelöst. Da die Ölkomponenten in den Reinigungsprodukten einen Film auf der Haut bilden können, ist es denkbar, dass die Wirkung der Tenside auf die hauteigenen Lipide und die natürlichen Feuchthaltefaktoren eingeschränkt ist.

Darüber hinaus bewirkt der Lipidfilm, dass die Haut nicht so stark austrocknet und trägt über die Rückfettung zur Regeneration des hauteigenen Lipidfilms bei.

Angegriffene Haut, sensible Haut oder Altershaut zeichnet sich häufig dadurch aus, dass sie trocken ist. Bei der Anwendung von üblichen Reinigungsprodukten, die kein Öl oder nur sehr wenig davon enthalten, wird durch das Herauslösen hauteigener Lipide und natürlicher Feuchthaltefaktoren eine weitere Beeinträchtigung der Haut bewirkt. Die Haut trocknet noch weiter aus. Es kann zu einem Spannungsgefühl der Haut, zu Rötungen und Juckreiz kommen. Gerade für Anwender mit derartigen Hautzuständen sind Reinigungszubereitungen auf Ölbasis in der Lage Abhilfe zu schaffen.

Im Stand der Technik wurden bereits seit längerer Zeit Reinigungszubereitungen auf Ölbasis offenbart.

Das Dokument DE 29 43 202 beschreibt Mittel mit reinigender und hautpflegender Wirkung auf der Basis von Gemischen aus Tensiden und Ölen, die bevorzugt als sogenannte Pflegeschaumbäder eingesetzt werden, wobei allerdings auch die Verwendung dieser Mittel als Duschzubereitung erwähnt wird. Die beschriebenen Zubereitungen haben einen Gehalt von 20 bis 80 Gew.-% einer wässrigen Tensidlösung, die ihrerseits aus 85 bis 95 Gew.-% Tensid und 5 bis 15 Gew.-% Wasser besteht, sowie einen Ölgehalt von 80 bis 20 Gew.-%. Die waschaktive Komponente dieser Zubereitungen besteht aus Mono-oder Dialkylamin-, Mono-oder Dialkanolamin-oder Alkylalkanolaminsalzen von Fettalkoholschwefelsäureestern.

Die Schrift EP 120 224 beschreibt wirkstoffhaltige Ölbadzubereitungen mit einem Gehalt an 38,75 Gew.-% Sojaöl, 2,00 Gew.-% Rizinusöl, 37,00 Gew.-% Vaselineöl. Als Emulgator werden Polyethylenglycolmono- bzw. -diester offenbart, in Konzentrationen von ca. 10 bis 12 Gew.-%. Der eingesetzte Wirkstoff ist Pelargonsäure.

Das Dokument DD 236014 offenbart ölhaltige kosmetische Mittel, die klar sein müssen. Diese Mittel finden u.a. Verwendung als Badeöle und Cremebäder. Neben 20 bis 80 % Öl, einer wässrigen Aniontensidlösung oder Wasser enthalten diese Zubereitungen eine Kombination aus bestimmten hydrophoben und hydrophilen Emulgatoren bzw. nichtionogenen Tensiden.

In dem Dokument DE 44 24 210 werden kosmetische oder dermatologische Duschzubereitungen mit einem Tensidgehalt von höchstens 55 Gew.-% und einem Ölgehalt von mehr als 45 Gew.-% beschrieben, wobei die Zubereitungen im Wesentlichen wasserfrei sind. Aufgrund des hohen Ölgehalts wirken diese Zubereitungen regenerierend in Bezug auf den allgemeinen Hautzustand. Sie haben dabei gleichzeitig eine gute Schaumentwicklung und eine hohe Reinigungskraft.

Die Schrift EP 0867176 beschreibt Duschzubereitungen, die einen hohen, jedoch im Vergleich zum Stand der Technik deutlich geringeren, Ölgehalt aufweisen. Es wird aber dennoch eine hohe Rückfettung erzielt bei besserer Schaumleistung und Verträglichkeit.

Das Dokument WO 03/051319 offenbart ebenfalls ölhaltige Reinigungsprodukte, in diesem Fall jedoch Reinigungsprodukte auf der Basis von ölhaltigen Mikroemulsionen. Es wird ein Verfahren zur Herstellung dieser ölhaltigen Mikroemulsionen beschrieben. Bei einer konstanten Gesamttensidmenge kann das Primärtensid-/Cotensid-Verhältnis variiert werden.

In dem Dokument WO 2005/065629 wird offenbart, dass ölhaltige, wasserfreie Tensidzubereitungen derart ausgestaltet werden können, dass sie einer Enzymschädigung der Enzyme der oberen Hautschichten entgegenwirken.

Im Dokument WO 2012/104025 werden auch lipidhaltige Badezubereitungen beschrieben, diese werden mit probiotisch aktiven Wirkstoffen versetzt.

Zur tiefergehenden Hauterfrischung und -reinigung gibt es Reinigungsmittel, die einen Peeling-Effekt aufweisen. Die peelende Wirkung kann dabei durch den Einsatz abrasiver Partikel zustande kommen. Bei den abrasiven Partikel handelt es sich in der Regel um natürliche oder synthetische, wasserunlösliche Feststoffe, in Form von Partikeln, die einen durchschnittlichen Durchmesser von 100 bis 400 µm und eine runde oder unrunde Gestalt aufweisen.

Im Rahmen einer Peelingbehandlung kann die abrasive Wirkung eingesetzter Partikel die Haut reizen. In Ausnahmefällen, insbesondere bei trockener und/oder empfindlicher Haut und/oder Altershaut kann es dadurch zu Irritationen kommen.

In der Vergangenheit hat es nicht an Versuchen gefehlt, dieser Reizung entgegen zu wirken, z. B. durch pflegende Additive, die die Haut beruhigen und/oder durch den Einsatz von Peelingpartikeln, die sich bei der Benutzung auflösen und somit einen abnehmenden Peelingeffekt zeigen.

Wasserlösliche Partikel, wie z. B. aus kristallbildenden Zuckern oder Salzen, können in mit diesen Zuckern oder Salzen übersättigte wässrige tensidhaltige Lösungen integriert werden. Derartige Lösungen sind aber schwer zu stabilisieren; die Partikel neigen dazu sich abzusetzen. Darüber hinaus fehlen solchen Formulierungen rückfettende Komponenten, die die Haut beruhigen und eine Regeneration bewirken können.

Unter dem Begriff Zucker können Mono- und Oligosaccharide (Dimere bis Decamere von Monosacchariden) verstanden werden, die süß schmecken, wasserlösliche und meist kristalline Verbindungen sind. Dem stehen die Polysaccharide gegenüber, die nur schlecht oder gar nicht in Wasser löslich sind, keine einheitliche Molmasse haben und praktisch geschmacksfrei sind.

Derartige Reinigungszubereitungen werden beispielsweise in den Dokumenten DE 102013224336 und DE 102013224368 beschrieben. Diese Zubereitungen sind Zubereitungen auf wässriger Basis, die keine oder nur geringe Mengen an Lipiden enthalten.

Das Dokument EP 1967173 A1 beschreibt Reinigungszubereitungen, die Reinigungskörper mit einer mittleren Korngröße von 100 bis 1000 µm enthaltend hydriertes Rizinusöl, Tenside und Verdicker enthalten. Eine Beispielrezeptur offenbart den Einsatz des Polyols Glycerin, jedoch in einem geringeren Anteil als in der vorliegenden Erfindung vorgesehen.

Im Dokument WO 2017/190989 A1 werden ebenfalls Reinigungszubereitungen beschrieben, die zwei Arten von abrasiven Partikeln enthalten. Alle Ausführungsbeispiele offenbaren einen hohen Wasseranteil, der deutlich über der erfindungsgemäß einzusetzenden Menge liegt.

Abrasive Partikel können auch direkt in lipidhaltige Zubereitungen eingearbeitet werden. Eine weitgehend pastöse Konsistenz wirkt dem Absinken dieser Partikel entgegen. Derartige Formulierungen lassen aber, selbst bei der Einarbeitung amphiphiler Komponenten, den vom Verbraucher gewünschten Schaum vermissen.

Es bestand also Bedarf, ölhaltige, weitgehend wasserfreie Reinigungszubereitungen zur Verfügung zu stellen, die eine tiefergehende Reinigung erzielen.

Weiterhin sollten die abrasiven Partikel, die den ölhaltigen, weitgehend wasserfreien Reinigungszubereitungen zugesetzt werden, weitgehend stabil in der Zubereitung verteilt sein und bleiben.

Bei der Anwendung der erfindungsgemäßen Zubereitungen sollte eine, mit üblichen Reinigungsprodukten vergleichbare Reinigungswirkung erzielt werden. Dies wird beispielsweise angezeigt durch eine angemessene Schaumbildung.

Um einen besonders reizarmen Peelingeffekt zu erzielen, ist es sehr vorteilhaft, wenn die Peelingpartikel sich zum überwiegenden Teil während der Benutzung auflösen und sich somit der Peelingeffekt im Verlaufe der Anwendung verringert.

Überraschend wurde nun gefunden, dass Reinigungszubereitungen auf der Basis einer Öl/Polyol Mischung, enthaltend abrasive Partikel und ein oder mehrere öllösliche Tenside, die vorstehenden Aufgaben zu lösen vermögen.

Es ist dabei von Vorteil, wenn die Ölphase ein gehärtetes Pflanzenöl oder eine Mischung verschiedener gehärteter Pflanzenöle und/oder -fette zur Strukturierung enthält, um die Stabilität der Zubereitung zu verbessern.

Weiterhin ist es wesentlich, wenn die Reinigungszubereitung auch polymere Strukturanten, insbesondere anionische Polymere enthält um eine Strukturierung zu bewirken.

Reinigungszubereitungen mit abrasiver Wirkung
mit einem Wassergehalt < 5,0 Gew.-%, bevorzugt < 1,5 Gew.-%, besonders bevorzugt < 0,2 Gew.-%, enthaltend
a. Lipide
b. Polyole
c. öllösliche Tenside
d. abrasive Partikel und
e. polymere Strukturanten,
wobei das Gewichtsverhältnis von Lipiden zu Polyolen 4 : 1 bis 1 : 4 beträgt,
wobei die Polyole Propylenglycol und/oder Glycerin sind,
wobei die polymeren Strukturanten vernetzte Acrylsäure- Copolymere sind, insbesondere Acrylsäure-Copolymere enthaltend Vinylmonomere, ganz besonders Acrylsäure - Copolymere enthaltend Vinylpyrrolidon-Monomere und
wobei die Lipide aus einer Mischung von bei Raumtemperatur flüssigen und festen Lipiden bestehen,
sind geeignet, die vorstehenden Aufgaben zu lösen.

Bevorzugt sind dabei Reinigungszubereitungen, die ein bestimmtes Gewichtsverhältnis bestimmter Komponenten aufweisen. Es ist bevorzugt, dass das Verhältnis der Summe der Gewichtsanteile aus Lipiden und Polyolen zu den Gewichtsanteilen aus Tensiden 2 : 1 bis 1 : 2 und bevorzugt 1,5 : 1 bis 1 : 1,5 beträgt.

Die Reinigungszubereitungen enthalten < 5,0 Gew.-%, bevorzugt < 1,5 Gew.-%, besonders bevorzugt < 0,2 Gew.-% Wasser. Der Wassergehalt kann mit Hilfe der Karl-Fischer-Titration bestimmt werden. Die Wasserbestimmung nach Karl Fischer ist geeignet um den Wassergehalt in organischen Lösungsmitteln, ätherischen Ölen, Salben und anderen organischen Substanzen zu bestimmen. Grundlage des Verfahrens ist die Tatsache, dass Jod und Schwefeldioxid nur in Gegenwart von Wasser zu Jodid und Sulfat reagieren. Der Endpunkt der Titration wird durch das Auftreten einer gelb-braunen Färbung angezeigt; an diesem Punkt ist das gesamte Wasser verbraucht und Jod reagiert nicht mehr zum farblosen Jodid. Heute wird diese Bestimmung mit vorbereiteten, käuflich zu erwerbenden Lösungen durchgeführt.

Die Reinigungszubereitungen enthalten Tenside, die öllöslich sind. Es hat sich als günstig erwiesen, wenn die Zubereitungen ein Haupttensid enthalten, das bevorzugt wenigstens ein nichtionisches Tensid ist, insbesondere ein Amid einer C₁₀ - C₁₆-Fettsäure, insbesondere bevorzugt ein Monoisopropylamid ist. Haupttenside im Sinne der vorliegenden Erfindung sind diejenigen Tenside, die unter Berücksichtigung aller Tenside in der erfindungsgemäßen Zubereitung, in der überwiegenden Menge vorliegen.

Neben dem Haupttensid können weitere Tenside vorliegen, die als Cotenside bezeichnet werden. Bevorzugt ist es, wenn wenigstens ein Cotensid ein weiteres Amid einer C₁₀ - C₁₆-Fettsäure ist. Dieses Amid einer C₁₀ - C₁₆-Fettsäure ist verschieden von dem als Haupttensid eingesetzten Amid einer C₁₀ - C₁₆-Fettsäure sein. Es ist besonders bevorzugt, wenn das Cotensid ein Diethanolamid ist.

Es hat sich ebenfalls als günstig erwiesen, wenn die Cotenside weitere, strukturell andersartige, nichtionische Tenside umfassen. Insbesondere bevorzugt werden als weitere nichtionische Tenside Tenside aus der Gruppe der alkoxylierten Fettalkohole mit einer Kettenlänge von 8 bis 16, bevorzugt 10 bis 14 C-Atomen und einem Alkoxylierungsgrad von 2 bis 10, bevorzugt 2 bis 6 ausgewählt. Der Alkoxylierungsgrad beschreibt die Anzahl der Alkoxygruppen im Molekül. Bevorzugte Alkoxygruppen sind Propylenoxid und Ethylenoxid. Besonders bevorzugt ist Ethylenoxid. Ein Ethylenoxid- Rest kann mit der Bezeichnung EO abgekürzt werden. Als Ethoxilierungsgrad wird die Anzahl der vorhandenen EO-Reste verstanden.

Es ist besonders bevorzugt, wenn die Cotenside Amide einer C₁₀ - C₁₆-Fettsäure und alkoxylierte Fettalkohole mit einer Kettenlänge von 8 bis 16, bevorzugt 10 bis 14 C-Atomen und einem Alkoxylierungsgrad von 2 bis 10, bevorzugt 2 bis 6 umfassen.

Es ist auch möglich, dass die Cotenside noch weitere nichtionische Tenside umfassen.

Der Gehalt an öllöslichen Tensiden beträgt 10 bis 60 Gew.-%, bevorzugt 20 bis 50 Gew.-%, insbesondere bevorzugt 25 bis 40 Gew.-%, bezogen auf das Gesamtgewicht der Zubereitung.

Die erfindungsgemäßen Lipide bestehen aus einer Mischung von bei Raumtemperatur flüssigen und festen Lipiden. Die bei Raumtemperatur flüssigen Lipide können synthetische und/oder natürliche, mineralische und/oder pflanzliche Öle sein. Es ist bevorzugt, wenn die flüssigen Lipide pflanzliche Öle sind, insbesondere bevorzugt Rizinus- und/oder Sojaöl und/oder Öl aus Sonnenblumenkernen.

Die bei Raumtemperatur festen Lipide können gehärtete Pflanzenöle und/oder -fette sein. Als Beispiele seien hier Hydrogenated Vegetable Oil, z. B. Dermofeel Viscolid der Firma Dr. Straetmans und Hydrogenated Vegetable Fat, z. B. VGB 22 der Firma ADM, genannt.

Der Gehalt an Lipiden in den erfindungsgemäßen Zubereitungen beträgt 5,5 bis 12,0 Gew.-%, bezogen auf das Gesamtgewicht der Zubereitung.

Das erfindungsgemäß bevorzugte Polyol ist Glycerin. Mit Hilfe der Polyole, insbesondere Glycerin, wird das Gesamtgewicht der erfindungsgemäßen Zubereitungen auf 100 Gew.-% gebracht. Es ist bevorzugt, wenn der Gehalt an Polyolen in einem Konzentrationsbereich von 5 bis 40 Gew.-%, bevorzugt 10 bis 27 Gew.-% liegt.

Die erfindungsgemäßen Reinigungszubereitungen enthalten polymere Strukturanten, die bevorzugt in die Mischphase aus Tensid(en) und Polyol(en) eingearbeitet werden. Die erfindungsgemäß bevorzugten Strukturanten sind anionische Polymere. Diese anionischen Polymere werden im Verlauf der Herstellung der erfindungsgemäßen Reinigungszubereitungen mit einer Base neutralisiert. Zur Neutralisierung des anionischen Polymers wird bevorzugt ein Amin eingesetzt, insbesondere bevorzugt Aminomethylpropanol.

Das anionische Polymer ist ein vernetztes Acrylsäure- Copolymer. Es ist weiter bevorzugt, wenn für die Synthese des Acrylsäure-Copolymers neben dem Monomer Acrylsäure als weiteres Monomer ein Vinylmonomer eingesetzt wird, insbesondere bevorzugt ein Vinylpyrrolidon-Monomer.

Der Gehalt an Strukturanten in den erfindungsgemäßen Zubereitungen beträgt 0,5 bis 3,0 Gew.-%, bevorzugt 0,8 bis 2,5 Gew.-%, bezogen auf das Gesamtgewicht der Zubereitung.

In den erfindungsgemäßen Reinigungszubereitungen sind als feste abrasive Partikel lösliche und/oder unlösliche Partikel enthalten.

Im Sinne der vorliegenden Erfindung werden unter festen abrasiven Partikeln, die löslich sind, Partikel verstanden, die in der Reinigungszubereitung im festen Aggregatzustand, insbesondere in Kristallform, vorliegen. Bei Anwendung der Reinigungszubereitung, beispielsweise beim Duschen, kommt die Reinigungszubereitung mit einer größeren Menge Wasser in Kontakt, so dass die Reinigungszubereitung verdünnt wird. Dies führt in der Regel dazu, dass sich die festen Partikel, insbesondere Kristalle, allmählich auflösen.

Die festen abrasiven Partikel, die löslich sind, werden bevorzugt aus Zuckern und/oder geeigneten und hautverträglichen Salzen generiert. Besonders bevorzugt ist der Einsatz von Saccharose und/oder Natriumchlorid zur Ausbildung der löslichen Partikel.

Die erfindungsgemäß einsetzbaren festen abrasiven Partikel, die unlöslich sind, haben einen synthetischen und/oder natürlichen Ursprung. Unlösliche Partikel natürlichen Ursprungs können sowohl mineralischen als auch pflanzlichen Ursprungs sein.

Die erfindungsgemäßen Reinigungszubereitungen können feste abrasive Partikel, die löslich sind, oder feste abrasive Partikel, die unlöslich sind, enthalten, sowie auch Mischungen löslicher und unlöslicher Partikel.

Ebenso Gegenstand der vorliegenden Erfindung ist die Verwendung von Reinigungszubereitungen mit abrasiver Wirkung
mit einem Wassergehalt < 5,0%, bevorzugt < 1,5%, besonders bevorzugt < 0,2%, enthaltend
a. Lipide
b. Polyole
c. öllösliche Tenside
d. abrasive Partikel und
e. polymere Strukturanten,
wobei das Gewichtsverhältnis von Lipiden zu Polyolen 4 : 1 bis 1 : 4 beträgt,
wobei die Polyole Propylenglycol und/oder Glycerin sind,
wobei die polymeren Strukturanten vernetzte Acrylsäure- Copolymere sind, insbesondere Acrylsäure-Copolymere enthaltend Vinylmonomere, ganz besonders Acrylsäure - Copolymere enthaltend Vinylpyrrolidon-Monomere und
wobei die Lipide aus einer Mischung von bei Raumtemperatur flüssigen und festen Lipiden bestehen,
zur tiefergehenden Reinigung bei trockener und/oder empfindlicher Haut und/oder Altershaut.

Folgende *Tenside* können in den erfindungsgemäßen Zubereitungen vorteilhaft verwendet werden:
Die erfindungsgemäß günstig zu verwendenden Amide der Fettalkoholsulfate bzw. der Fettalkoholethersulfate weisen folgende Struktur auf:

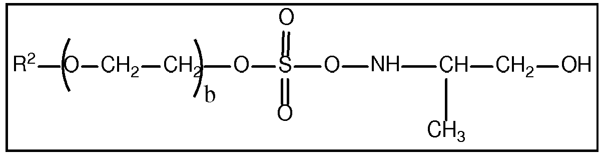

Dabei kann b Werte von 0 bis 10, vorteilhaft 1 bis 5 annehmen. R² wird gewählt aus der Gruppe der verzweigten und unverzweigten Alkylgruppen mit 6 bis 24 Kohlenstoffatomen.

Bevorzugtes Fettalkoholethersulfat ist MIPA-Laurethsulfat.

Vorteilhaft weisen die erfindungsgemäß günstig zu verwendenden Fettalkoholethoxylate folgende Struktur auf:

R³-(O-CH₂-CH₂-)_{c}-OH

Dabei kann c Werte von 2 bis 10 annehmen, bevorzugt von 2 bis 6. R³ wird gewählt aus der Gruppe der verzweigten und unverzweigten Alkylgruppen mit 8 bis 16 Kohlenstoffatomen, insbesondere 10 bis 14 Kohlenstoffatome.

Bevorzugtes Fettalkoholethoxylat ist Laureth-4.

Vorteilhaft weisen die erfindungsgemäß günstig zu verwendenden Fettsäuremono- bzw. - diethanolamide folgende Strukturen auf:

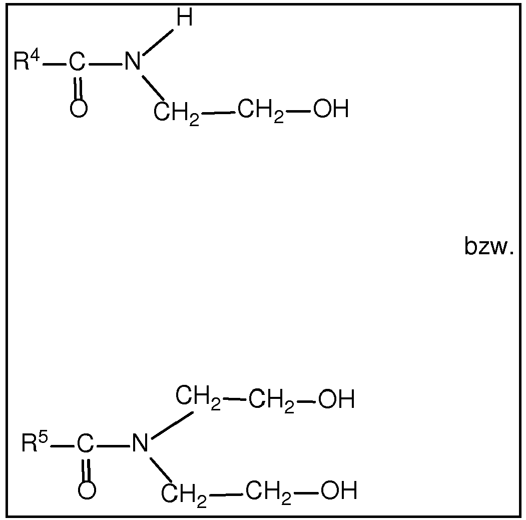

R⁴ bzw. R⁵ werden dabei gewählt aus der Gruppe der verzweigten und unverzweigten Alkylgruppen und/oder Alkenylgruppen mit 6 bis 24 Kohlenstoffatomen, insbesondere mit 10 bis 16 Kohlenstoffatomen.

Bevorzugtes Fettsäurediethanolamid ist Kokosfettsäurediethanolamid (Cocamide DEA). Natürliche Kokosfettsäure enthält als wesentliche Bestandteile Laurinsäure zu 44-51 Gew.-%, Myristinsäure zu 13-18 Gew.-%, Palmitinsäure zu 8-10 Gew.-%, Caprylsäure zu 6-9 Gew.-%, Caprinsäure zu 6-10 Gew.-%, Ölsäure zu 5- 8 Gew.-%, Stearinsäure zu 1-3 Gew.-%, Linolsäure zu 0-2 Gew.-% und Capronsäure zu 0-1 Gew.-%.

Ganz besonders bevorzugt ist, Gemische aus MIPA-Laurethsulfat, Laureth-4 und Kokosfettsäurediethanolamid einzusetzen. Solche Gemische sind beispielsweise unter der Bezeichnung ZETESOL® 100 von der Firma Zschimmer & Schwarz Chemische Fabriken, Lahnstein/Rhein, oder TEXAPON® WW 99 von der Henkel KGaA, Düsseldorf, erhältlich.

Die erfindungsgemäßen *Öle* werden vorzugsweise gewählt aus der Gruppe der Triglyceride folgender Struktur: 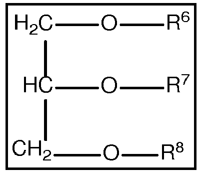 wobei R⁶, R⁷ und R⁸ unabhängig voneinander gewählt werden aus der Gruppe der verzweigten und unverzweigten, Alkylcarboxyl bzw. Alkenylcarboxylgruppen mit 12 bis 24 Kohlenstoffatomen. Es ist gegebenenfalls vorteilhaft, wenn eine oder mehrere aliphatische Wasserstoffatome der Alkylcarboxyl- bzw. Alkenylcarboxylgruppen durch Hydroxylgruppen substituiert sind.

Für die Auswahl der bei Raumtemperatur flüssigen Lipide ist es insbesondere vorteilhaft, wenn R⁶, R⁷ und/oder R⁸ 16 bis 20 Kohlenstoffatome aufweisen und aus der Gruppe der einfach bis dreifach ungesättigten Carbonsäurereste gewählt werden.

Wenn R⁶, R⁷ und/oder R⁸ Hydroxylgruppen tragen, ist der bevorzugte Alkenylcarboxylrest der Ricinolsäurerest.

Besonders vorteilhaft ist, die erfindungsgemäßen bei Raumtemperatur flüssigen Lipide aus der Gruppe Sojaöl, Rizinusöl und Sonnenblumenkernöl auszuwählen.

Für die Auswahl der bei Raumtemperatur festen Lipide ist es vorteilhaft, wenn diese einen Schmelzpunkt oder Schmelzbereich in einem Fenster von 50 bis 70ºC, eine Jodzahl zwischen 0 und 5 und einen Gehalt an freien Fettsäuren von 0 bis 0,8 Gew.-% haben.

*Polyole* in Sinne der vorliegenden Erfindung sind mehrwertige Alkohole. Es handelt sich um niedermolekulare Verbindungen, die mindestens zwei Hydroxylgruppen tragen. Erfindungsgemäß sind die Verbindungen Propylenglycol und Glycerin, bevorzugt ist Glycerin.

*Feste abrasive Partikel* in Sinne der vorliegenden Erfindung sind lösliche und/oder unlösliche Partikel, die eine Peelingwirkung hervorrufen.

Bei den löslichen Partikeln wird die Peelingwirkung durch den ungelösten Anteil an Zuckern oder Salzen, der in kristalliner Form vorliegen kann, hervorgerufen. Dies wird durch eine so hohe Zucker- und/oder Salzkonzentration erreicht, dass sich nicht alle Zucker- und/oder Salzmoleküle in der erfindungsgemäßen Zubereitung lösen können, sondern in fester bzw. kristalliner Form vorliegen. Diese so gearteten Peelingsubstanzen kommen bei Anwendung während des Dusch- oder Waschvorgangs mit Wasser in Kontakt, beispielsweise beim Abduschen oder Abwaschen des Schaums und der Reinigungszubereitung am Ende des Reinigungsvorgangs. Hierdurch ist ein Auflösen der Zucker- und/oder Salzkristalle gewährleistet, was zu einem abnehmenden Peelingeffekt führt. Dies führt aber gleichzeitig auch dazu, dass keine oder praktisch keine Rückstände der Peelingsubstanzen in Waschbecken oder Behandlungsvorrichtungen zurückbleiben. Praktisch keine Rückstände bedeutet, dass in einzelnen wenigen Fällen äußerst geringe Spuren der Reinigungszubereitung nach dem Waschvorgang auf Waschtischen oder Anwendungseinrichtungen sichtbar sind, die jedoch auf einfache Weise durch Abspülen oder Wegwischen entfernbar sind.

Die erfindungsgemäß einsetzbaren Zucker können beispielsweise Mono-, Di- oder Oligosaccharide sein. Mono- oder Disaccharide können beispielsweise Saccharose, Glukose oder Fruktose sein, wobei Saccharose ganz besonders bevorzugt ist.

Die Salze in den erfindungsgemäßen Zubereitungen sollen hautverträglich und im Milieu der Reinigungszubereitungen geeignet sein, feste Strukturen, bevorzugt Kristalle zu bilden. Dies können beispielsweise Alkalisalze sein, wie Natriumchlorid, Kaliumchlorid, Natriumsulfat und/oder Kaliumsulfat, wobei Natriumchlorid ganz besonders bevorzugt ist. Die Salze in den erfindungsgemäßen Zubereitungen können ebenfalls Erdalkalisalze sein, wie beispielsweise Magnesiumchlorid und/oder Magnesiumsulfat.

Die unlöslichen Peelingmittel können beispielsweise vorteilhaft aus der Gruppe Polyethylen, Tonerde, Sand, Kunststoffpartikel, zerstoßene oder zermahlene Kerne von Walnussschalen, Aprikosen-, Pfirsich- und/oder Mandelkernen gewählt werden.

Die erfindungsgemäß einsetzbaren polymeren Strukturanten sind anionische Polymere. Erfindungsgemäß sind anionische Polymere, die Acrylsäure als Monomer aufweisen. Es sind beispielsweise Polyacrylate wie Acrylat-Alkylacrylat-Copolymere, insbesondere solche, die aus der Gruppe der sogenannten Carbomere gewählt werden.

Erfindungsgemäß bevorzugt können bei der Synthese der anionischen Polymere zusätzlich Vinylmonomere eingesetzt werden, insbesondere bevorzugt Vinylpyrrolidon.

Erfindungsgemäß besonders bevorzugt sind Verbindungen mit der INCI-Bezeichnung Acrylic Acid/VP Crosspolymer.

Um die Wirksamkeit der erfindungsgemäßen Zubereitungen zu belegen, wurde eine ausgewählte Zubereitung, OELPEEL:70, in einem In-Use-Screening eingesetzt. Die Zubereitung OELPEEL:70 wurde an 100 Probandinnen verteilt, die das Produkt mindestens viermal innerhalb von 12 Tagen benutzen mussten. Es wurde ein Fragebogen ausgeteilt, mit dem verschiedene Produktleistungen abgefragt wurden. Von 100 Fragebögen wurden 83 zurückgegeben und konnten ausgewertet werden. Die Probandinnen waren alle weiblich, im Schnitt 37,08 Jahre alt. Alle Probandinnen waren Verwenderinnen von Duschpeelingprodukten. Die Fragen konnten auf einer Skala von 1 (stimme nicht zu) bis 7 (stimme zu) beantwortet werden. Die Antworten wurden in Bezug auf die Ermittlung des jeweiligen Mittelwertes, der absoluten und relativen Häufigkeit ausgewertet. Zusätzlich wurden sogenannte Topboxes und Lowboxes ermittelt, d.h. die Antworten in Bezug auf jeweils besten und zweitbesten bzw. die schwächsten und zweit schwächsten Aussagen gruppiert.

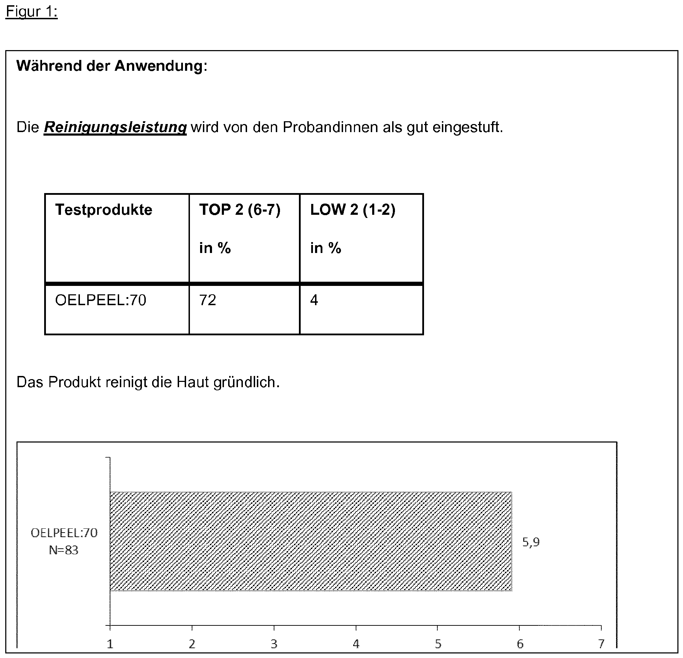

Die Reinigungsleistung der Zubereitung OELPEEL:70 wurde während der Anwendung beurteilt, 36,4 % der Probandinnen stimmten zu und zwar mit dem höchst möglichen Wert von 7 (siehe Figur 1). Die Reinigungsleistung von OELPEEL:70 wird insgesamt als außerordentlich gut bewertet.

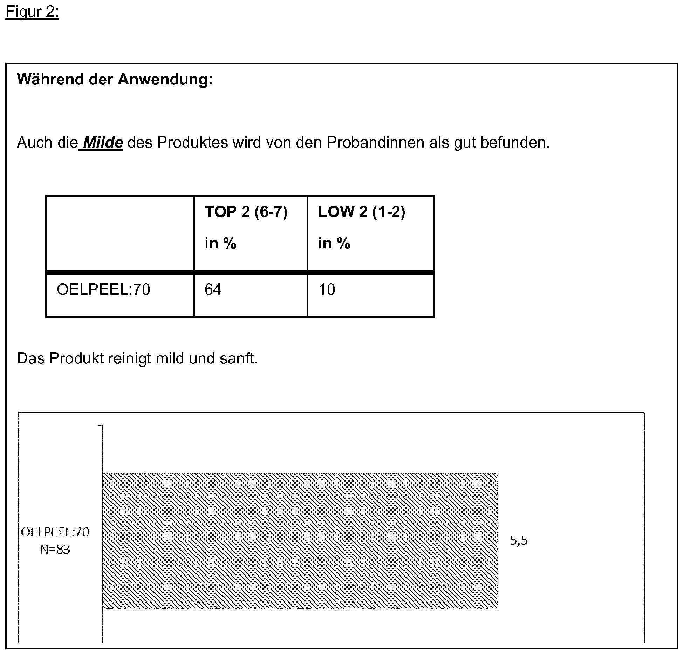

Ebenso wurde die Milde während der Anwendung beurteilt, 28,7 % der Probandinnen bewerteten die Milde mit den höchst möglichen Wert von 7, 32,2 % mit einem Wert von 6, den zweit höchsten Wert. Die Zubereitung OELPEEL:70 weist eine ausgezeichnete Milde auf, siehe Figur 2.

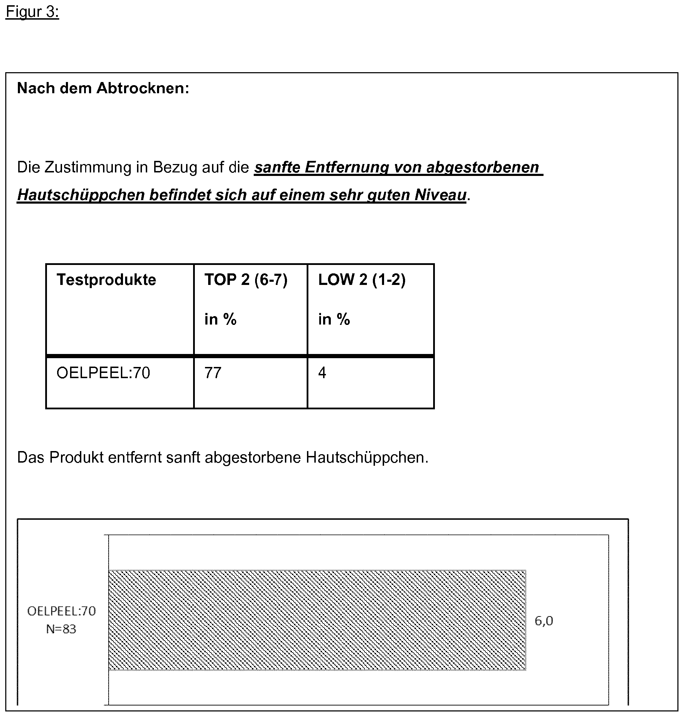

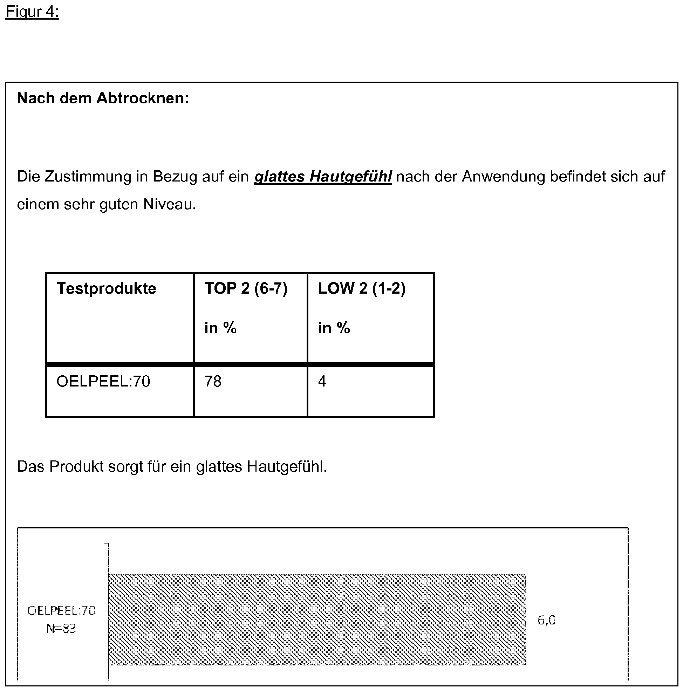

Nach dem Abtrocknen sollten die Probandinnen eine Beurteilung zur sanften Entfernung von Hautschüppchen, zum Hautgefühl und zur Austrocknung der Haut abgeben, siehe Figuren 3 bis 6). 47,2 % der Probandinnen bewerteten die Entfernung der Hautschüppchen mit dem höchsten Wert von 7. Daraus folgt, dass die Peelingwirkung durch die Zuckerkristalle als sehr gut bewertet wurde.

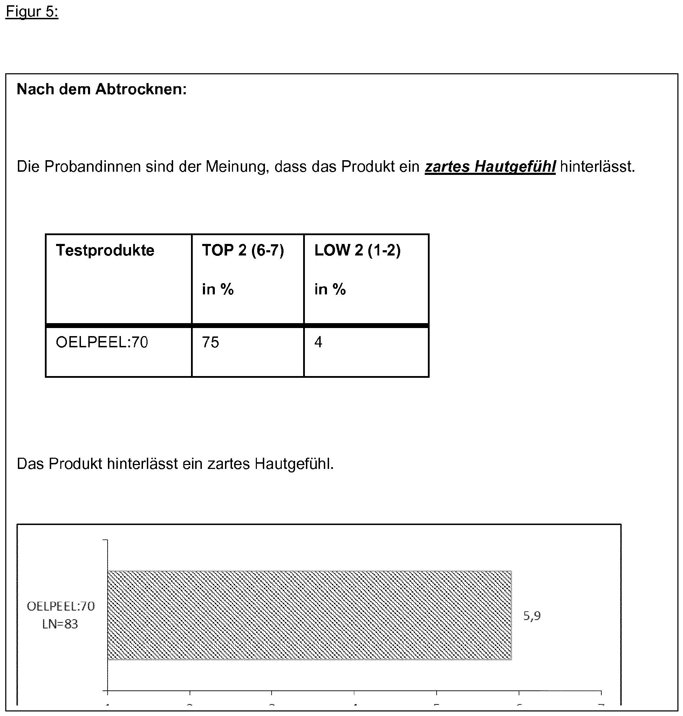

Dass das Hautgefühl in Bezug auf Glätte und Zartheit ausgezeichnet ist, wird ebenfalls mit hohen Werten beurteilt (Figur 5).

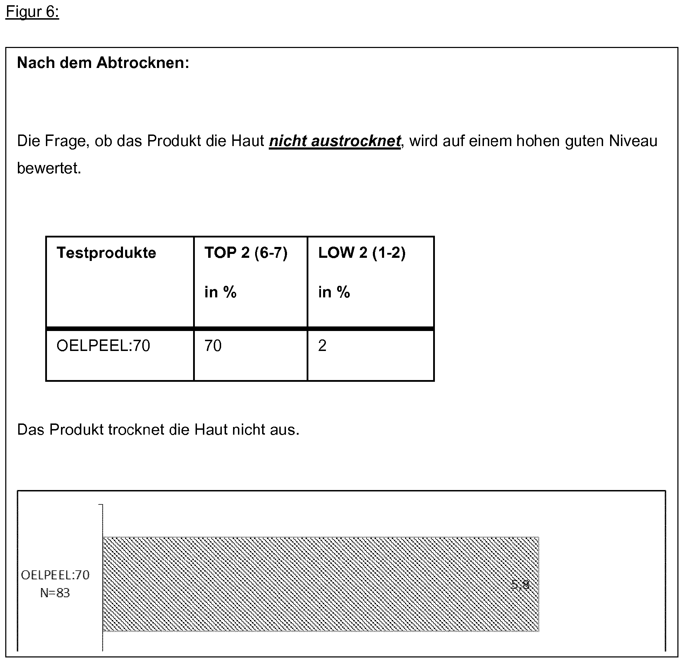

34,1 % der Probandinnen stimmten mit dem höchsten Wert zu, dass das Produkt die Haut nicht austrocknet (Figur 6).

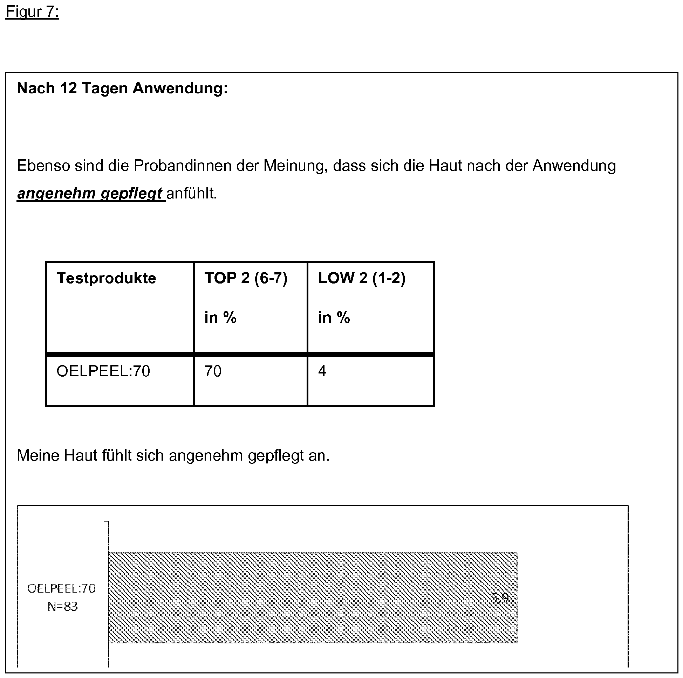

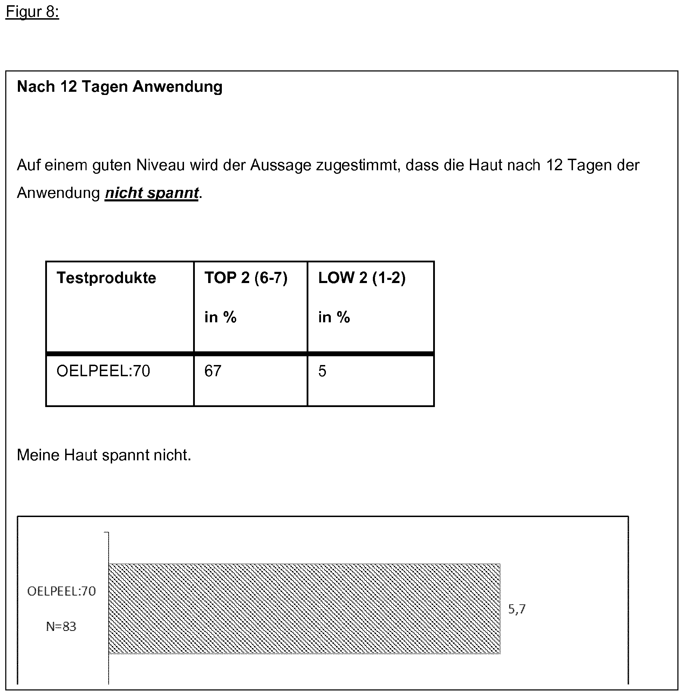

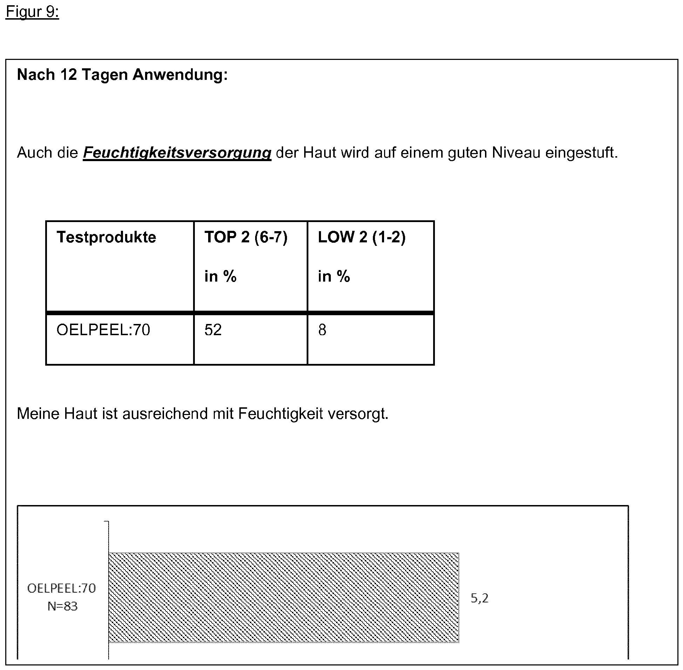

Nach 12 Tagen Anwendung wurden die Probandinnen zu der Pflege, zu einem Spannungsgefühl der Haut und zur Feuchtigkeitsversorgung der Haut befragt, siehe Figur 7 bis 9. Alle drei Aspekte wurden von den Probandinnen als überdurchschnittlich gut beurteilt.

Das in dem IN-USE-Screening verwendete Produkt, OELPEEL:70, hat folgende Zusammensetzung:

| **Inci** | **Handelsname Hersteller** | |
|---|---|---|
| Acrylic Acid/VP Crosspolymer | Ultrathix P-100 Ashland | 1,1 |
| MIPA Laureth Sulfate + Laureth-4 + Cocoamide DEA | Zetesol 100 Zschimmer & Schwarz | 35,0 |
| Glycerine + Aqua | Glycerine 99,5% BP EmeryOleochemicals | Ad. 100% |
| Aminomethyl Propanol + Aqua | AMP 95 Angus Chemie | 0,3 |
| Ricinus Communis Seed Oil | Rizinusoel Gustav Heess | 5,0 |
| Glycine Soja Oil + Ricinus Communis Seed Oil + Propyl Gallate | Sojaöl, Gustav Heess | 5,0 |
| BHT | Ionol CP; Art. 1240 Oxiris Chemicals | 0,05 |
| Sucrose | Sandzucker Nordzucker AG | 30,0 |
| Hydrated Silica | Sipernat 2200 Evonik Industries | 5,0 |
| Hydrogenated Vegetable Oil | Dermofeel Viscolid Dr. Straetmanns | 1,0 |
| Fragrance | Parfum | 1,0 |

### Beispiele:

Alle Angaben in der vorliegenden Anmeldung sind Angaben in Gew.-%, sofern nicht ausdrücklich andere Einheiten angegeben sind.

Die Angaben in den unten stehenden Beispiele geben die Rohstoffgehalte an. Diese entsprechen den Aktivgehalten, sofern nicht im Handelsnamen der jeweilige prozentuale Aktivgehalt angegeben ist.

| **Inci** | **Handelsname Hersteller** | | 1 | 2 | 3 | 4 | 5 |
|---|---|---|---|---|---|---|---|
| Acrylic Acid/VP Crosspolymer | Ultrathix P-100 Ashland | | 1,2 | 1,3 | 1,0 | 1,5 | 1,3 |
| MIPA Laureth Sulfate + Laureth-4 + Cocoamide Dea | Zetesol 100 Zschimmer & Schwarz | | 25,5 | 30,0 | 35,0 | 30,0 | 40,0 |
| Glycerine + Aqua | Glycerine 99,5% BP EmeryOleochemicals | | Ad. 100% | Ad. 100% | Ad. 100% | Ad. 100% | Ad. 100% |
| Aminomethyl Propanol + Aqua | AMP 95 Angus Chemie | | 0,25 | 0,3 | 0,3 | 0,2 | 0,25 |
| Ricinus Communis Seed Oil | Rizinusoel Art Nr. 300031 Gustav Hess | | 5,0 | 5,0 | 4,5 | 2,5 | 3,0 |
| Helianthus Annuus Seed Oil | Sonneblumenoel raff. Henry Lamotte | | 3,0 | 5,0 | 5,0 | 5,0 | 1,0 |
| BHT | Ionol CP; Art. 1240 Oxiris Chemicals | | 0,05 | 0,06 | 0,05 | 0,04 | 0,00 |
| Sucrose | Sandzucker Nordzucker AG | | 35,0 | 30,0 | 30,0 | 20,0 | 25,0 |
| Hydrated Silica | Sipernat 2200 Evonik Industries | | 6,0 | 4,0 | 4,7 | 8,0 | 3,5 |
| Propylene Glycol | Propylene Glycol USP/EP-RMT Dow Chemical | | 0,0 | 2,0 | 0,0 | 5,0 | 0,0 |
| Hydrogenated Vegetable Oil | Dermofeel viscolid Dr. Straetmans | | 0 | 0,5 | 1,2 | 0,7 | 0 |
| Hydrogenated Vegetable Fat | VGB 22 ADM | | 1,5 | 0,5 | 0 | 0,6 | 1,9 |
| Fragrance | Parfum | | 1 | 1,1 | 1,0 | 1,2 | 1,0 |

## Patentansprüche

1. Reinigungszubereitungen mit abrasiver Wirkung
mit einem Wassergehalt < 5,0%, bevorzugt < 1,5%, besonders bevorzugt < 0,2%, enthaltend
a) Lipide
b) Polyole
c) öllösliche Tenside
d) abrasive Partikel und
e) polymere Strukturanten,
wobei das Gewichtsverhältnis von Lipiden zu Polyolen 4 : 1 bis 1 : 4 beträgt,
wobei die Polyole Propylenglycol und/oder Glycerin sind,
wobei die polymeren Strukturanten vernetzte Acrylsäure- Copolymere sind, insbesondere Acrylsäure-Copolymere enthaltend Vinylmonomere, ganz besonders Acrylsäure -Copolymere enthaltend Vinylpyrrolidon-Monomere und
wobei die Lipide aus einer Mischung von bei Raumtemperatur flüssigen und festen Lipiden bestehen.

2. Reinigungszubereitungen nach Anspruch 1, **dadurch gekennzeichnet, dass** das Verhältnis der Summe der Gewichtsanteile aus Lipiden und Polyolen zu den Gewichtsanteilen aus Tensiden 2 : 1 bis 1 : 2 und bevorzugt 1,5 : 1 bis 1 : 1,5 beträgt.

3. Reinigungszubereitungen nach Anspruch 1 und/oder 2, **dadurch gekennzeichnet, dass** der Gehalt an Polyolen in einem Konzentrationsbereich von 5 bis 40 Gew.-%, bevorzugt 10 bis 27 Gew.-% liegt.

4. Reinigungszubereitungen nach wenigstens einem der vorstehenden Ansprüche, **dadurch gekennzeichnet, dass** der Gehalt an öllöslichen Tensiden 10 bis 60 Gew.-%, bevorzugt 20 bis 50 Gew.-%, insbesondere bevorzugt 25 bis 40 Gew.-%, bezogen auf das Gesamtgewicht der Zubereitung, beträgt.

5. Reinigungszubereitungen nach wenigstens einem der vorstehenden Ansprüche, **dadurch gekennzeichnet, dass** die öllöslichen Tenside Gemische aus MIPA-Laurethsulfat, Laureth-4 und Kokosfettsäurediethanolamid sind.

6. Reinigungszubereitungen nach wenigstens einem der vorstehenden Ansprüche, **dadurch gekennzeichnet, dass** der Gehalt an Lipiden in den erfindungsgemäßen Zubereitungen 5,5 bis 12,0 Gew.-%, bezogen auf das Gesamtgewicht der Zubereitung, beträgt.

7. Reinigungszubereitungen nach wenigstens einem der vorstehenden Ansprüche, **dadurch gekennzeichnet, dass** die bei Raumtemperatur flüssigen Lipide Rizinus- und/oder Sojaöl und/oder Öl aus Sonnenblumenkernen sind.

8. Reinigungszubereitungen nach wenigstens einem der vorstehenden Ansprüche, **dadurch gekennzeichnet, dass** die bei Raumtemperatur festen Lipide Hydrogenated Vegetable Oil und/oder Hydrogenated Vegetable Fat sind.

9. Reinigungszubereitungen nach wenigstens einem der vorstehenden Ansprüche, **dadurch gekennzeichnet, dass** feste abrasive Partikel enthalten sind, wobei die festen abrasiven Partikel lösliche oder unlösliche Partikel sind.

10. Reinigungszubereitungen nach Anspruch 9, **dadurch gekennzeichnet, dass** die löslichen Partikel aus Zuckern oder Salzen bestehen.

11. Reinigungszubereitungen nach Anspruch 10, **dadurch gekennzeichnet, dass** die löslichen Partikel aus Saccharose bestehen.

12. Reinigungszubereitungen nach Anspruch 10, **dadurch gekennzeichnet, dass** die löslichen Partikel aus Natriumchlorid bestehen.

13. Reinigungszubereitungen nach Anspruch 9, **dadurch gekennzeichnet, dass** die unlöslichen Partikel synthetischen oder natürlichen Ursprungs oder Mischungen von Partikeln synthetischen oder natürlichen Ursprungs sind.

14. Verwendung von Reinigungszubereitungen gemäß Anspruch 1 zur tiefergehenden Reinigung bei trockener und/oder empfindlicher Haut und/oder Altershaut.

## Claims

1. Cleansing preparations with abrasive effect
having a water content of <5.0%, preferably <1.5%, particularly preferably <0.2%, comprising
a) lipids
b) polyols
c) oil-soluble surfactants
d) abrasive particles and
e) polymeric structurants,
wherein the ratio by weight of lipids to polyols is from 4:1 to 1:4,
wherein the polyols are propylene glycol and/or glycerol,
wherein the polymeric structurants are crosslinked acrylic acid copolymers, particularly acrylic acid copolymers comprising vinyl monomers, especially acrylic acid copolymers comprising vinylpyrrolidone monomers and
wherein the lipids consist of a mixture of lipids that are liquid and solid at room temperature.

2. Cleansing preparations according to Claim 1, **characterized in that** the ratio of the sum of the proportions by weight of lipids and polyols to the proportions by weight of surfactants is from 2:1 to 1:2 and preferably from 1.5:1 to 1:1.5.

3. Cleansing preparations according to Claim 1 and/or 2, **characterized in that** the content of polyols is in a concentration range of 5 to 40% by weight, preferably 10 to 27% by weight.

4. Cleansing preparations according to at least one of the preceding claims, **characterized in that** the content of oil-soluble surfactants is from 10 to 60% by weight, preferably 20 to 50% by weight, particularly preferably 25 to 40% by weight, based on the total weight of the preparation.

5. Cleansing preparations according to at least one of the preceding claims, **characterized in that** the oil-soluble surfactants are mixtures of MIPA-laureth sulfate, laureth-4 and coconut fatty acid diethanolamide.

6. Cleansing preparations according to at least one of the preceding claims, **characterized in that** the content of lipids in the preparations according to the invention is from 5.5 to 12.0% by weight, based on the total weight of the preparation.

7. Cleansing preparations according to at least one of the preceding claims, **characterized in that** the lipids that are liquid at room temperature are castor oil and/or soybean oil and/or oil from sunflower seeds.

8. Cleansing preparations according to at least one of the preceding claims, **characterized in that** the lipids that are solid at room temperature are hydrogenated vegetable oil and/or hydrogenated vegetable fat.

9. Cleansing preparations according to at least one of the preceding claims, **characterized in that** solid abrasive particles are present, wherein the solid abrasive particles are soluble or insoluble particles.

10. Cleansing preparations according to Claim 9, **characterized in that** the soluble particles consist of sugars or salts.

11. Cleansing preparations according to Claim 10, **characterized in that** the soluble particles consist of sucrose.

12. Cleansing preparations according to Claim 10, **characterized in that** the soluble particles consist of sodium chloride.

13. Cleansing preparations according to Claim 9, **characterized in that** the insoluble particles are of synthetic or natural origin or mixtures of particles of synthetic or natural origin.

14. Use of cleansing preparations according to Claim 1 for deep cleansing of dry and/or sensitive skin and/or ageing skin.

## Revendications

1. Préparations nettoyantes à action abrasive, ayant une teneur en eau < 5,0 %, de préférence < 1,5 %, de manière particulièrement préférée < 0,2 %, contenant :
a) des lipides,
b) des polyols,
c) des tensioactifs solubles dans les huiles,
d) des particules abrasives et
e) des structurants polymères,
le rapport en poids entre les lipides et les polyols étant de 4:1 à 1:4,
les polyols étant le propylène glycol et/ou la glycérine,
les structurants polymères étant des copolymères d'acide acrylique réticulés, notamment des copolymères d'acide acrylique contenant des monomères de vinyle, tout particulièrement des copolymères d'acide acrylique contenant des monomères de vinylpyrrolidone, et
les lipides étant constitués par un mélange de lipides liquides et solides à température ambiante.

2. Préparations nettoyantes selon la revendication 1, **caractérisées en ce que** le rapport entre la somme des proportions en poids de lipides et de polyols et les proportions en poids de tensioactifs est de 2:1 à 1:2, et de préférence de 1,5:1 à 1:1,5.

3. Préparations nettoyantes selon la revendication 1 et/ou 2, **caractérisées en ce que** la teneur en polyols se situe dans une plage de concentration allant de 5 à 40 % en poids, de préférence de 10 à 27 % en poids.

4. Préparations nettoyantes selon au moins l'une quelconque des revendications précédentes, **caractérisées en ce que** la teneur en tensioactifs solubles dans les huiles est de 10 à 60 % en poids, de préférence de 20 à 50 % en poids, de manière particulièrement préférée de 25 à 40 % en poids, par rapport au poids total de la préparation.

5. Préparations nettoyantes selon au moins l'une quelconque des revendications précédentes, **caractérisées en ce que** les tensioactifs solubles dans les huiles sont des mélanges de MIPA-laureth sulfate, de laureth-4 et de diéthanolamide d'acide gras de coco.

6. Préparations nettoyantes selon au moins l'une quelconque des revendications précédentes, **caractérisées en ce que** la teneur en lipides dans les préparations selon l'invention est de 5,5 à 12,0 % en poids, par rapport au poids total de la préparation.

7. Préparations nettoyantes selon au moins l'une quelconque des revendications précédentes, **caractérisées en ce que** les lipides liquides à température ambiante sont l'huile de ricin et/ou de soja et/ou l'huile de graines de tournesol.

8. Préparations nettoyantes selon au moins l'une quelconque des revendications précédentes, **caractérisées en ce que** les lipides solides à température ambiante sont une huile végétale hydrogénée et/ou une graisse végétale hydrogénée.

9. Préparations nettoyantes selon au moins l'une quelconque des revendications précédentes, **caractérisées en ce que** des particules abrasives solides sont contenues, les particules abrasives solides étant des particules solubles ou insolubles.

10. Préparations nettoyantes selon la revendication 9, **caractérisées en ce que** les particules solubles sont constituées par des sucres ou des sels.

11. Préparations nettoyantes selon la revendication 10, **caractérisées en ce que** les particules solubles sont constituées par du saccharose.

12. Préparations nettoyantes selon la revendication 10, **caractérisées en ce que** les particules solubles sont constituées par du chlorure de sodium.

13. Préparations nettoyantes selon la revendication 9, **caractérisées en ce que** les particules insolubles sont d'origine synthétique ou naturelle ou des mélanges de particules d'origine synthétique ou naturelle.

14. Utilisation de préparations nettoyantes selon la revendication 1 pour le nettoyage approfondi de la peau sèche et/ou sensible et/ou de la peau mature.
